(19) Europäisches Patentamt
European Patent Office
Office européen des brevets

(11) **EP 3 893 756 B1**

(12) **EUROPEAN PATENT SPECIFICATION**

(45) Date of publication and mention of the grant of the patent:
**14.08.2024 Bulletin 2024/33**

(21) Application number: **19829685.7**

(22) Date of filing: **13.12.2019**

(51) International Patent Classification (IPC):
**A61B 5/1455** (2006.01)   **A61B 3/12** (2006.01)
**A61B 3/10** (2006.01)   **A61B 5/083** (2006.01)
**A61B 5/00** (2006.01)

(52) Cooperative Patent Classification (CPC):
**A61B 5/0833; A61B 3/10; A61B 3/12;**
**A61B 5/14555; A61B 5/4845**

(86) International application number:
**PCT/IB2019/060775**

(87) International publication number:
**WO 2020/121276 (18.06.2020 Gazette 2020/25)**

(54) **POINT-OF-CARE (POC) DEVICE FOR RAPID DIAGNOSIS OF POISONING**

VORRICHTUNG FÜR DEN EINSATZ AM BEHANDLUNGSORT ZUR SCHNELLEN DIAGNOSE VON VERGIFTUNGEN

DISPOSITIF DE POINT DE SERVICE (PDS) POUR DIAGNOSTIC RAPIDE D'EMPOISONNEMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.12.2018 IT 201800011113**

(43) Date of publication of application:
**20.10.2021 Bulletin 2021/42**

(73) Proprietor: **Università degli Studi di Bari "Aldo Moro"**
**70121 Bari (BA) (IT)**

(72) Inventors:
- **SANTACROCE, Luigi**
  **70121 Bari (IT)**
- **CHARITOS, Ioannis Alexandros**
  **70121 Bari (IT)**
- **NANNA, Giuseppe**
  **70121 Bari (IT)**

(74) Representative: **Fiume, Orazio**
**Praxi Intellectual Property S.p.A.**
**Corso Vittorio Emanuele II, 3**
**10125 Torino (IT)**

(56) References cited:
WO-A2-02/26119   WO-A2-2010/143208
CN-A- 102 028 477   US-A- 4 877 322
US-A1- 2018 336 679

- **JONA VALGERDUR KRISTJANSDOTTIR ET AL: "Choroidal Oximetry With a Noninvasive Spectrophotometric Oximeter", INVESTIGATIVE OPTHALMOLOGY & VISUAL SCIENCE, vol. 54, no. 5, 7 May 2013 (2013-05-07), US, pages 3234, XP055606311, ISSN: 1552-5783, DOI: 10.1167/iovs.12-10507**
- **ASBJORG GEIRSDOTTIR ET AL: "Retinal Vessel Oxygen Saturation in Healthy Individuals", INVESTIGATIVE OPTHALMOLOGY & VISUAL SCIENCE, vol. 53, no. 9, 13 August 2012 (2012-08-13), US, pages 5433, XP055606315, ISSN: 1552-5783, DOI: 10.1167/iovs.12-9912**
- **LAING R A ET AL: "The choroidal eye oximeter: an instrument for measuring oxygen saturation of choroidal blood in vivo", IEEE TRANSACTIONS ON BIOMEDICAL ENGINEERING, IEEE SERVICE CENTER, PISCATAWAY, NJ, USA, vol. BME-22, no. 3, 1 May 1975 (1975-05-01), pages 183 - 195, XP002200046, ISSN: 0018-9294**

EP 3 893 756 B1

**Description**

Technical Field

The present invention relates to a device for the rapid diagnosis of poisoning by enzymatic toxic substances (such as cyanide) in a human subject, by virtue of their ability to inhibit the use of the oxygen coming from capillary blood by mitochondrial cellular systems.

Prior Art

Oxygen saturation is a blood index that reflects the percentage of oxygen-saturated haemoglobin with respect to the total amount of haemoglobin present in the blood.

In physiological conditions, during breathing, with the passage into the lungs, the red blood cells rich in haemoglobin are saturated with oxygen, which will then be transported and transferred to the various tissues through the capillaries of the organism.

Via the complexes of the mitochondrial respiratory chain, the cells use blood-derived oxygen to produce energy (in the form of ATP). Cyanide performs its own toxic action by binding to one of the components of the mitochondrial respiratory chain, the cytochrome c, blocking a fundamental step of cellular metabolism. The cell, therefore, normally receives oxygen but is not able to use it for its own needs, as the mitochondrial systems of ATP production are blocked. The venous blood leaving the tissues, instead of being deprived of a portion of oxygen used by the cells, will remain with an unchanged oxygen load: for this reason, the oxygen content of the venous blood will correspond to that of the arterial blood, as there has been no consumption by "intoxicated" cells, i.e. blocked in enzymatic energy systems. In the organism intoxicated with cyanide, this results in normal blood oxygen levels (normal arterial saturation) without extraction by the cells and therefore abnormal venous saturation (higher than normal).

The measurement of oxygen saturation, therefore, constitutes the parameter that reflects the percentage of oxygen molecules linked to haemoglobin (oxyhaemoglobin), and allows to determine possible states of hypoxemia (reduced amount of $O_2$ available in the blood).

Oxygen saturation is typically measured with a transcutaneous electromedical instrument, called saturometer (oximeter or pulse oximeter) having a shape similar to that of a clip.

This device is equipped with a probe and two light-emitting diodes (sensors emitting light rays of different wavelengths and communicating with a photocell). The blood index is then evaluated by means of the absorption of the light emitted by the saturometer applied to a finger of the hand or a lobe of the ear (anatomical regions rich in capillaries).

Arterial oxygen saturation is considered normal when the values are above 95%, while it begins to become pathological if equal to or lower than 90%.

This device has the disadvantage of not being adequately sensitive to abnormal saturation of haemoglobin in the veins.

The methods currently used are invasive (laboratory blood tests in hospital) and have long response times, for example, they require procedures and equipment that are generally not available on site, subtracting time from the timely diagnosis of patient intoxication and immediate life-saving antidote treatment.

To the knowledge of the inventors, there are currently no devices based on non-invasive measurement of oxygen saturation in the retinal veins that are portable, do not require treatment with mydriatic substances and do not require an external data-elaborating system, such as a computer.

CN102028477A (Institute of Optics and Electronics, Chinese Academy of Sciences, 27th April 2011) describes a device capable of acquiring images of the illuminated retina with different wavelengths (680nm, 796nm). Such images can then be analysed with the help of an external computer to calculate the oxygen saturation in the blood. However, such device is not portable, requires intervention by the operator (who, among other things, must select the points of the image in which to calculate the oxygen saturation) and external hardware for processing. Therefore, it is not suitable for use in emergency situations.

US 2018/336679 A1 (Richard M. Farchione et al., 22nd November 2018) describes a fundus portable camera capable of acquiring images of the retina. Such images should then be examined by medical personnel to diagnose eye diseases, such as diabetic retinopathy. Such device acquires a single image of the same portion of the illuminated fundus oculi with different wavelengths at the same time and does not perform measurements or diagnoses autonomously.

JONA VALGERDUR KRISTJANSDOTTIR ET AL: "Choroidal Oximetry With a Noninvasive Spectrophotometric Oximeter", INVESTIGATIVE OPTHALMOLOGY & VISUAL SCIENCE, vol. 54, no. 5, 7 May 2013 (2013-05-07), page 3234, XP055606311, US ISSN: 1552-5783, DOI: 10.1167/iovs.12-10507 discloses a device for determining the oxygenation level in retinal vessels by illuminating and imaging the fundus at wavelengths of 570 nm and 600 nm, respectively, calculating respective optical densities as a logarithm of a ratio between the intensity of the pixels detected in a proximity of a section of veins and arteries in said images and the intensity of the pixels detected on said section itself, and calculating a ratio of the optical densities at the two wavelengths to determine the level of oxygenation.

Summary of the Invention

It is the object of the invention to develop a portable and easy-to-use device for the rapid diagnosis of intoxication with toxic enzymatic substances such as cyanide, capable of interfering with the oxygen utilization by tissues, consequently causing a serious tissue hypoxic condition despite apparently normal levels of arterial blood saturation and which can occur in emergency/urgency situations.

In physiological conditions, a drop in blood oxygen saturation is measurable through a classic saturometer. This can happen in case of respiratory failure, cardiac arrest, foreign bodies in the airways. Blood carrying less oxygen reaches the cells and this causes their suffering and, sometimes, necrosis.

In the case of cyanide intoxication, however, oxygen is normally present in the blood, but the cells are no longer capable of using it. Hypoxia always occurs but with a different mechanism (in the first case lack of oxygen, in the second inability of cells of using it).

The device of the invention allows to measure the oxygen saturation in the venous vessels of the retina of a patient likely to be intoxicated by enzymatic toxic substances such as cyanide, emitting a positive signal when this results to be abnormally high compared to physiological values. The finding of a high venous saturation means, in practical terms, that the cells have not been able to extract it from the incoming arterial blood. The diagnosis of tissue hypoxia is currently possible through a blood test that reveals specific haematochemical parameters, in particular, elevated blood levels of lactic acid linked to the activation of anaerobic metabolism of cells in conditions of oxygen unavailability, and the gradual drop in the blood pH towards a condition of acidosis. These analyses need time and can often be performed in a hospital setting or can exclusively be interpreted by trained personnel.

The device, on the other hand, allows an immediate diagnosis at the site of intoxication (domestic, industrial, woodland, impervious, confined) even by non-medical personnel as long as they are trained in the interpretation of the result on the instrument.

Since the retinal vessels are a mirror of what happens into the vessels of the whole body of the intoxicated patient but with the advantage of being visible in the front line without intervening tissues interfering with a direct visual analysis (as happens in the classic saturometers, able to measure arterial blood saturation but not venous saturation, due to the presence of skin and subcutaneous tissue interposed between vessels and instrument), their visibility can be exploited through the eye to measure directly the chromatic variation linked to the different oxygen saturation of haemoglobin, in particular by analysing the anomalous venous saturation which, in the intoxicated patient, is high while it should be below a certain range.

The innovation of the present invention relates to the ability to reveal a condition of tissue hypoxia which would not otherwise be visible with a classic saturometer; the latter, actually, positioned on an intoxicated patient's fingertip, would measure "normal" blood saturation values (e.g. 95%) but would not be able to reveal the cellular suffering that is occurring in the meantime due to the inability of the cells to use oxygen. This diagnostic delay leads the patient to develop a severe lactic acidosis (as the cell metabolism, deprived of oxygen, changes toward anaerobic metabolism) with severe consequences for the whole organism that result in death. The device of the invention allows to highlight the chromatic variations of the retinal vessels due to the high level of oxygen saturation in the venous blood, correlating them to the state of acute poisoning and intoxication of a subject.

The device of the invention is provided with an eyepiece which, resting on the periorbital area of the patient, through a lens for indirect ophthalmoscopy, acquires by means of a camera the pictures of the ocular fundus, illuminated alternately with LEDs having two distinct wavelengths.

Through the picture analysis, a computer built into the device measures oxygen saturation in the retinal blood vessels and communicates it to the operator. In particular, the device is able to distinguish between veins and arteries.

Other objects will become apparent from the following description of the invention and in particular from the dependent claims, which form an integral part of the present description.

Brief Description of the Figures

The characteristics and advantages of the invention will become apparent from the following detailed description, given with reference to the attached drawings, provided purely by way of non-limiting example, in which:

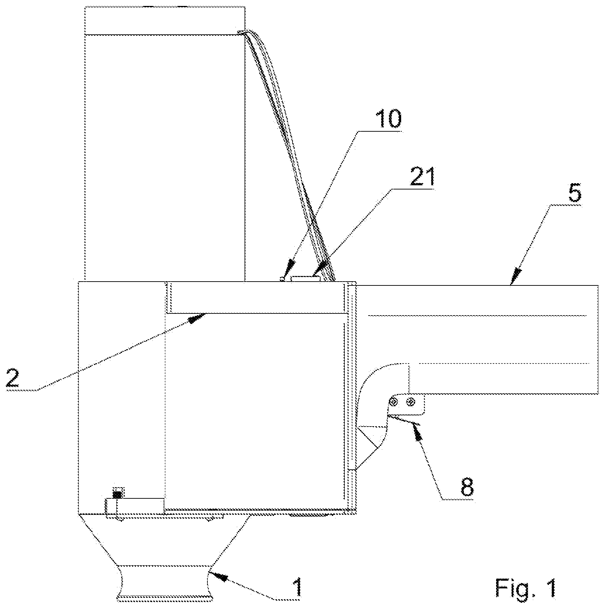

Figure 1 is a schematic left side view of the device of the invention;

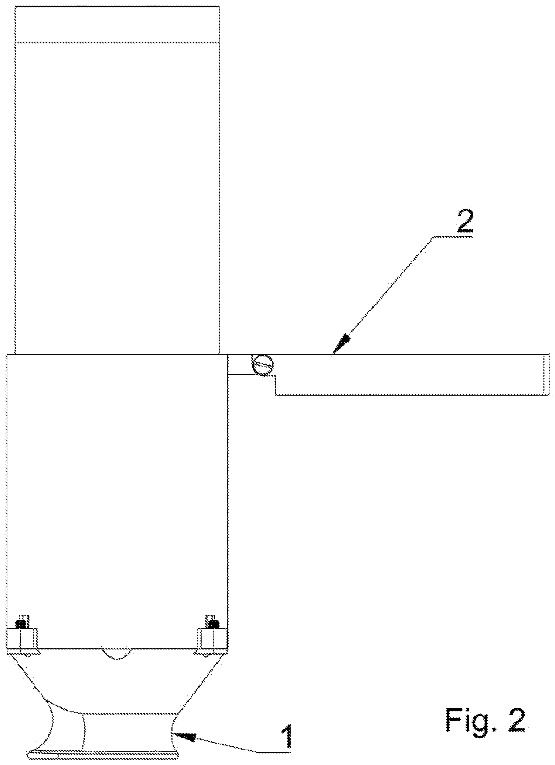

Figure 2 is a schematic front side view of the device of the invention;

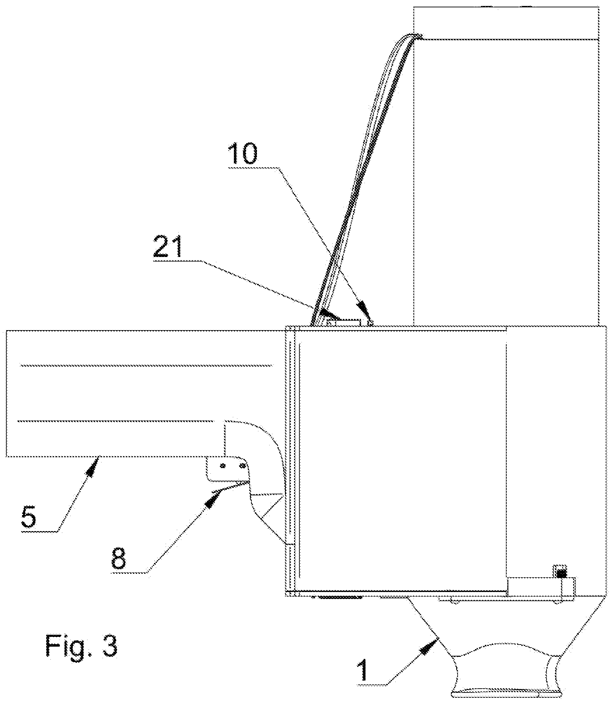

Figure 3 is a schematic right-side view of the device of the invention;

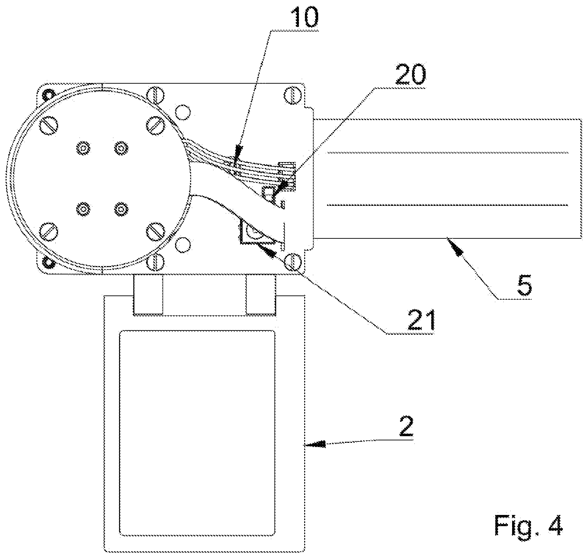

Figure 4 is a schematic front top view of the device of the invention;

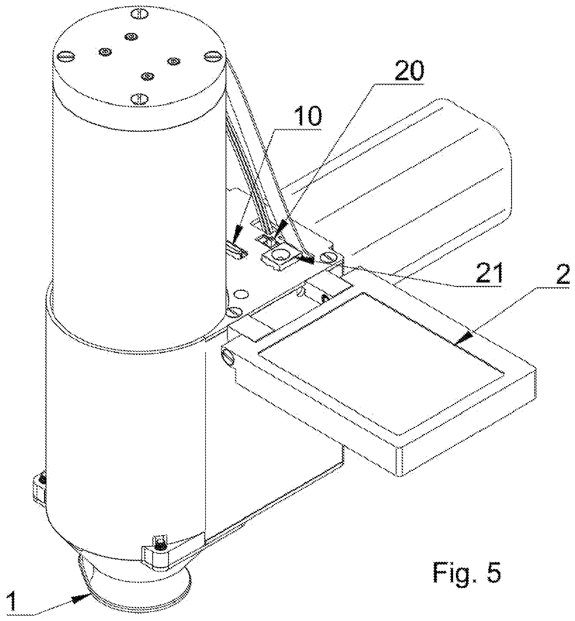

Figure 5 is a schematic isometric view of the device of the invention;

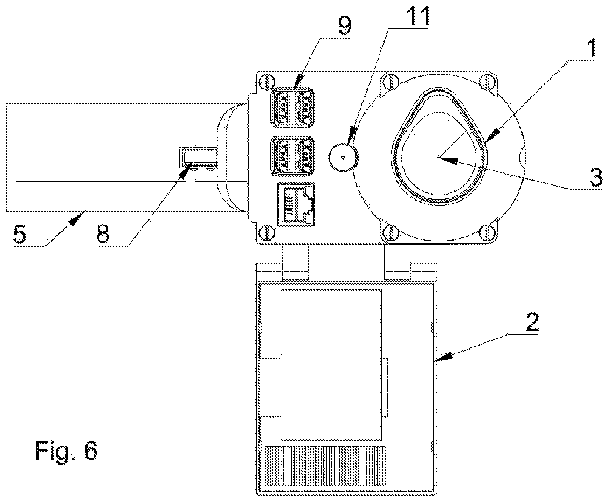

Figure 6 is a schematic front bottom view of the device of the invention;

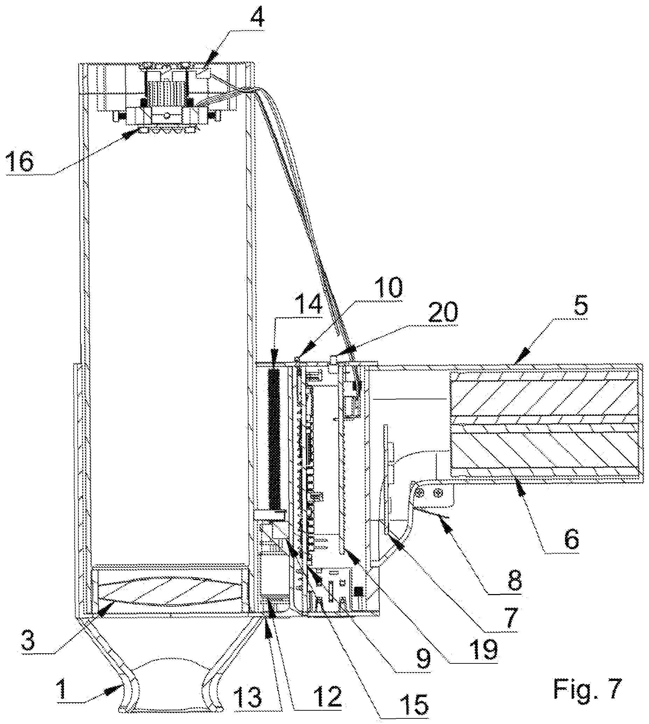

Figure 7 is a schematic view in sagittal section of the device of the invention;

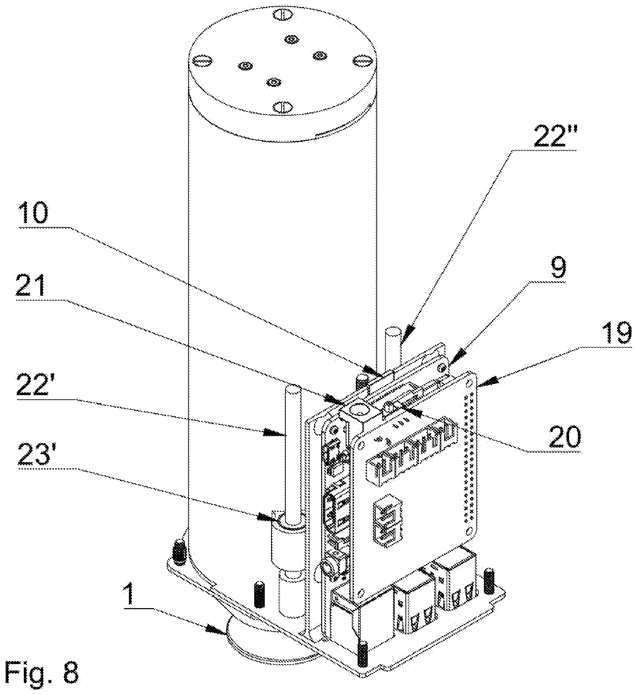

Figure 8 is a schematic isometric view of the internal part of the device;

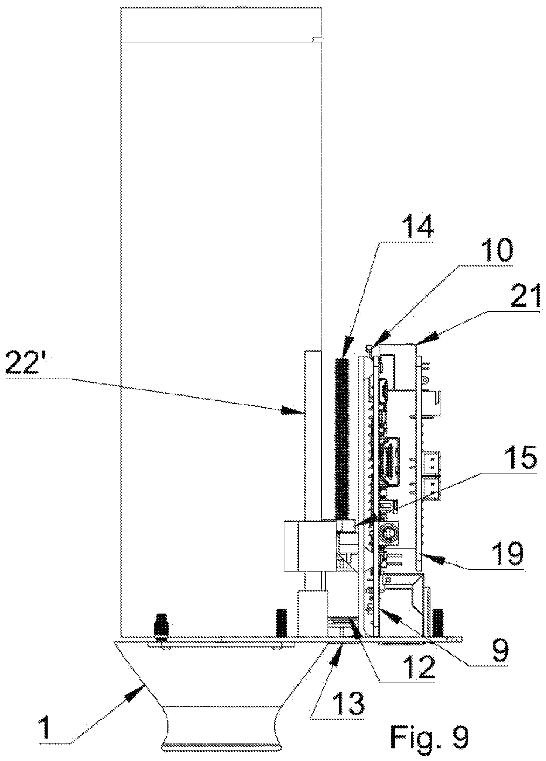

Figure 9 is a schematic left side view of the internal part of the device;

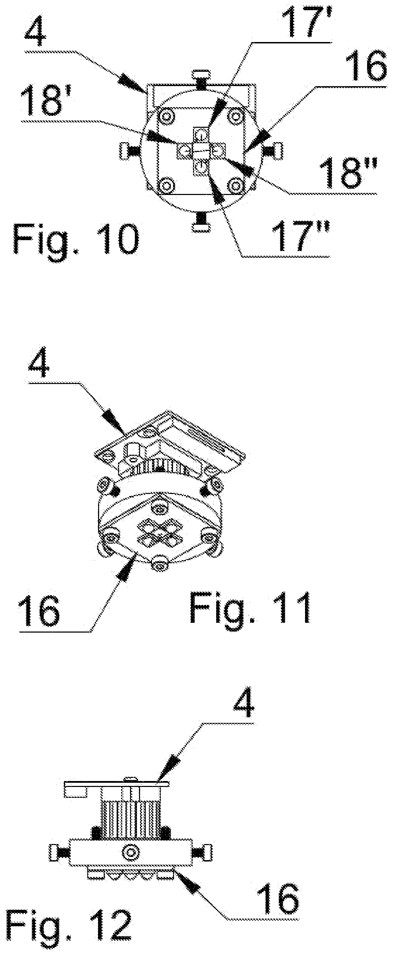

Figure 10 is a schematic front bottom view of the camera and the LEDs holder of the device of the invention;
Figure 11 is a schematic isometric view of Figure 10;
Figure 12 is a schematic front view of the camera and the LEDs holder of the device of the invention;

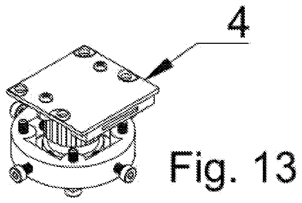

Figure 13 is a schematic top isometric view of the camera and the LEDs holder of the device of the invention.

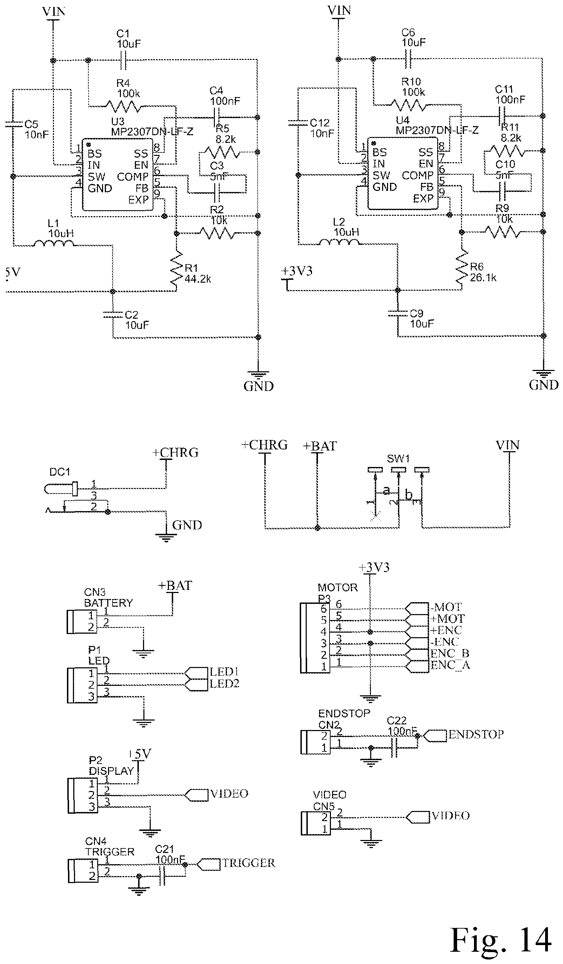

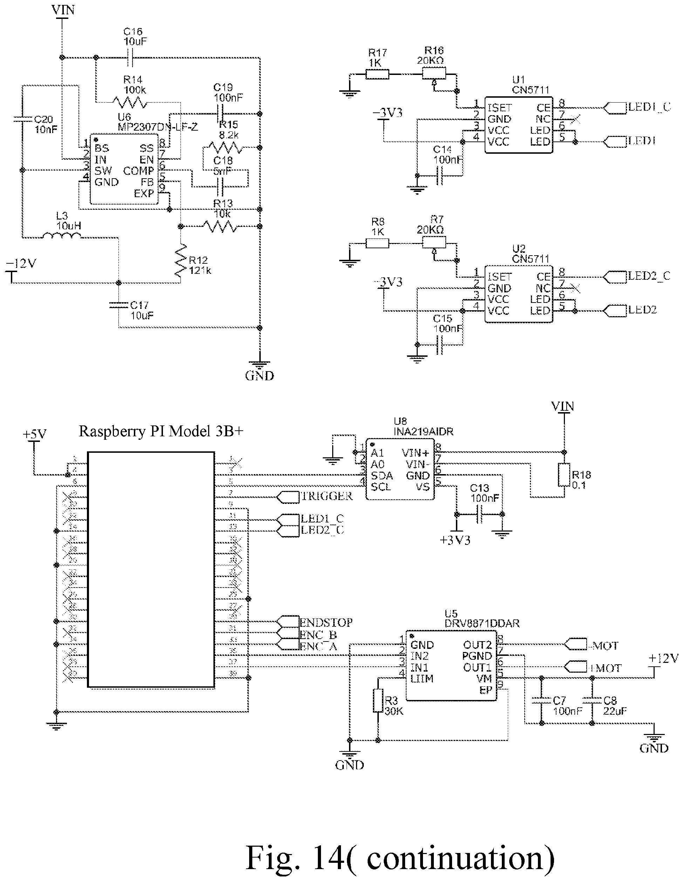

Figure 14 is the wiring diagram of the second printed circuit board (19) of the device of the invention.

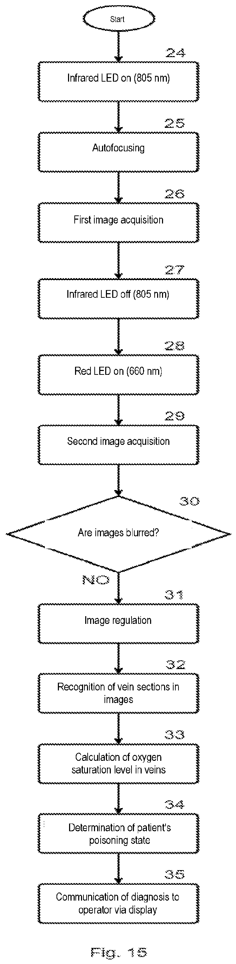

Figure 15 is a flow diagram describing an exemplary operation of the device of the invention.

Detailed Description

The present invention is based on the experimentation conducted from which it was found that in subjects in states of poisoning and consequent acute cyanide poisoning, immediate chromatic variations of the retinal vessels due to the high level of oxygen saturation in the venous blood, i.e. oxyhaemoglobin, were found. This is due to the action of the $CN^{(-)}$ ion which blocks the mitochondrial respiration chain and prevents the body from using oxygen from oxyhaemoglobin.

The basic elements of the device are:

- a camera sensitive to the infrared spectrum (i.e. without an IR filter) with a peak wavelength of 660 nm (red);
- one or more light sources, such as emission diodes or high-power LEDs, with a peak wavelength equal to 805 nm (infrared);
- a computer built into the device for the analysis of the pictures obtained by means of the camera;
- a lens for indirect ophthalmoscopy;
- a casing or housing or case that prevents external light from reaching the camera;
- a display.

The device is also equipped with a button switching the device on and off and a power source, such as a battery.

The device of the invention, of limited size and weight, therefore portable, preferably comprises a small single-board computer, which operates two pairs of LEDs having a different frequency (805 nm and 660 nm) and is connected to a camera module sensitive to the visible and infrared spectrum.

The camera is placed behind a 20-dioptre lens and a mobile motorized eyepiece, capable of moving the lens-camera system away from or closer to the eye to be examined (for autofocus).

A non-transparent, i.e. opaque, plastic casing encloses all components and isolates the camera from external light sources.

Since this is a mobile device, there is a module that manages the charging and discharging of a lithium battery that in turn supplies all the elements of the device that require electricity to operate. Finally, there are buttons for switching the device on and off and a small display, which constitute the operator interface.

The LEDs are located on the sides of the camera. The LEDs and the camera are pointed toward the ophthalmoscopy lens, which is positioned at such a distance that the camera can focus on the image produced by the lens. A rubber eyepiece is located on the other side of the lens. Such components are enclosed in a casing in such a way that, once the eyepiece is placed on the periocular area of the individual being examined, the light from external sources is prevented from reaching the eye or camera.

The device allows the immediate acquisition of data relating to any anomalies in oxygenation in the retinal veins of the patient. The aim is a rapid differential diagnosis (within one minute) and without the intervention of an ophthalmologic specialist for systemic toxic asphyxiating substances such as hydrocyanic acid-based compounds. These substances block the mitochondrial respiration chain by blocking the enzyme cytochrome a3 (cit a3) or Warburg's respiratory ferment, thus leading to tissue hypoxia that is potentially lethal.

In practice: once the eyepiece (1) has been placed on the patient's periorbital area, the measurement is started by pressing the button (8). Initially (24) infrared LEDs (18', 18") are lit at 805 nm. Since they do not produce visible light, they do not cause the pupil to contract.

In the embodiment of the device, the output of the camera (4) is shown in real time on the display (2) to allow the operator to centre the patient's eye.

In this step, the focusing operations (25) are performed, which in the proposed embodiment correspond to bringing the set consisting of the lens (3) and the camera (4) to the distance from the patient's eye that minimizes the blur of the images acquired by the camera. The amount of blur can be estimated, for example, by calculating the variance of Laplace (Pech-Pacheco, J.L., et al. (2000). Diatom Autofocusing in Brightfield Microscopy: a Comparative Study. ICPR. http://doi.org/10.1109/ICPR.2000.903548).

Then, the camera takes two pictures of the ocular fundus in rapid succession (26, 27, 28, 29), firstly illuminating the fundus only with the infrared LEDs at 805 nm (18', 18"), secondly illuminating the fundus only with the red LEDs at 660 nm (17', 17"). Turning on the red LEDs and acquiring the second picture shall occur within 230 ms, time (O. Bergamin el al. Invest. Ophthalmol. Vis. Sci. 2003; 44(4):1546-1554. http://doi.org/10.1167/iovs.02-0468) beyond which the pupil reacts to the light stimulus by contracting.

If (30) one of the images should not result sharp due to blur caused by the movement of the eye or device, the test procedure should be interrupted and, if necessary, repeated.

Since the image processing step requires that the pictures belong to the same portion of the retina and that the blood vessels are in the same positions in both pictures, both images shall be adjusted (31) using a linear transformation model.

An algorithm (Kim H., et al. (2011) Differentiation of Artery and Vein in Digital Fundus Photograph. In: Jobbágy Á. (eds) 5th European Conference of the International Federation for Medical and Biological Engineering. IFMBE Proceedings, vol 37. Springer, Berlin, Heidelberg https://doi.org/10.1007/978-3-642-23508-5 149) applied to the image acquired with red light, based on the analysis of the intensity profile of circular areas around the optical disc, allows to identify the positions of retinal vessels on the image. In particular, it identifies (32) a list of sections of vessels and classifies them into sections of veins or arteries. Since both pictures are acquired in rapid succession and realigned, the sections identified by this method on the picture acquired with red light have the same coordinates also on the other picture.

As described in the document by Jóna Valgerður Kristjánsdóttir (1985) Choroidal and retinal oximetry, http://hdl.handle.net/1946/17137 at pages 20-23, for each of the two pictures and for each vessel section previously identified, an approximation of the absorbance OD is calculated as the logarithm of the ratio between the intensity $I_0$ of the pixels sampled in proximity, that is, belonging to the immediate vicinity, of the given vessel section and the intensity I of the pixels sampled on the same section.

$$OD = log\ (I_0/I)$$

The expression "in the immediate vicinity" means that pixels not belonging to the vessel section but resulting adjacent to the pixels relative to the vessel section are analysed.

The $I_0/I$ ratio is an approximation of the ratio of incident light intensities emerging from the observed sample. In particular, $I_0$ is proportional to the intensity of the light that does not interact with the blood into the vessels. I is proportional to the intensity of the light reflected by the vessels, which has been partially absorbed by the blood. (Jóna Valgerður Kristjánsdóttir (1985) Choroidal and retinal oximetry, http://hdl.handle.net/1946/17137)

The absorbance values, measured on the picture taken with infrared LEDs (as opposed to the photo taken with red LEDs), do not depend on the oxygen saturation in the blood, i.e. the frequency of 805 nm is isosbestic (Oxyhaemoglobin and deoxyhaemoglobin have (not exclusively) isosbestic points at 586 nm and near 808 nm. https://en.wikipedia.org/wiki/Isosbestic_point).

For each of the sections, the ODR ratio of the absorbances at the two wavelengths is calculated

$$ODR = OD_{660}/OD_{805}$$

The amount of ODR depends linearly on the oxygen saturation in the blood $SatO_2$, i.e.:

$$SatO_2 = a\ *\ ODR + b$$

The arithmetic mean of the oxygen saturations of the vein sections is then calculated (33).

Since in healthy individuals oxygen saturation in the retinal veins is around 55% (Thorunn S. et al. Invest. Ophthalmol. Vis. Sci.2012;53(14):2172 https://doi.org/10.1167/iovs.12-9912), significantly higher values allow to indicate a low level of oxygenation as, for example, resulting from cyanide poisoning (34).

The outcome of the diagnosis will be communicated (35) to the operator via the display.

Description of a Preferred Embodiment of the Invention

With particular reference to the attached figures, the device according to the invention includes a non-transparent cylindrical casing, preferably made of plastic, which encloses the following elements:

- a rubber eyepiece (1);
- an output device configured for displaying information, or display (2);
- an aspherical magnifying lens for indirect ophthalmoscopy (3);
- an infrared camera (4), i.e. one camera, the only one, capable of acquiring both visible and ultra-red light;
- a rechargeable battery pack (5) consisting of four lithium-ion batteries (6) and the corresponding electronic card (7) configured as a battery management system;
- a touch button (8) configured to start the measurement;
- a single-board computer (9), wherein a removable storage device, in particular, an SD memory card (10), is installed;
- a linear actuator (11) consisting of an electric motor (12), a rotary magnetic encoder (13), a threaded rod (14) and a limit switch (15), and configured to move away/closer the set consisting of the lens (3) and said infrared camera (4) to the eye of the patient to be examined;
- a pair of linear guides (22', 22") and linear ball bearings (23', 23") thereof, which allow the movement of the set consisting of the lens (3) and the infrared camera (4);
- a first printed circuit board (16) comprising a pair of red LEDs at 660 nm (17', 17") and a pair of infrared LEDs at 805 nm (18', 18");
- a second printed circuit board (19) installed on the computer (9), for which the following wiring diagram is provided, configured for:

    o routing of the connections between the computer (9) and the LEDs (17', 17", 18', 18"), the linear actuator (11), the display (2) and the battery pack (5),
    o power distribution, comprising a slide switch (20) configured to connect/disconnect the battery pack to the printed circuit board, and a connector (21) for charging,
o LEDs control (17', 17", 18', 18"),
o linear actuator control (11),
o measurement of the residual charge of the battery pack (5).

The operation of the device according to the invention is described hereinbelow.

The battery pack (5) is connected to connector CN3 of the second printed circuit board (19). On such printed circuit board there is the switch SW1 (20), which connects/disconnects the battery pack to the printed circuit board. The connector DC1 (21) is connected directly to the battery pack so that it can be recharged using an external power supply.

The integrated circuit INA219 (U8) allows the measurement of the battery pack voltage so that it is possible to estimate its residual charge, which is shown on the display (2).

The integrated circuit DRV8871 allows the computer RPI1 (9) to control the linear actuator (11), connected to connector P3, and the limit switch (15) thereof is connected to connector CN2.

The linear actuator (11) is fixed to a plastic cylinder, the magnifying lens (3) being fixed to the first end of said cylinder and the camera (4) being fixed at the second end of said cylinder. The linear actuator (11) allows to move away/closer the set consisting of lens (3) and camera (4).

The integrated circuits CN5711 (U1) (U2) allow the computer (9) to control the pair of red LEDs (17', 17") and the pair of infrared LEDs (18', 18"), respectively.

The integrated circuits MP2307 (U3) (U4) (U4) lower the input voltage to 5 V, 3.3 V and 12 V respectively, to supply the computer (9), the linear actuator (11), the LEDs (17", 17', 18', 18") and the display (2) in accordance with the wiring diagram provided hereinbelow.

The video signal coming from the computer (9) is sent to the display (2), connected to the connector P2. On the other hand, the camera (4) is connected directly to the computer (9).

The display (2) shows in real time the images coming from the camera (4) to allow the operator to centre the patient's eye. Furthermore, the display (2) shows, at the end of the procedure, the results of the processing of the acquired images carried out by the analysis programs installed on the single-board computer (9). In order to reduce the overall dimensions, the display (2) can be folded laterally on the device by means of a hinge.

The micro touch button (8) is connected to connector CN4 of the second card (19). The measurement is started by the operator pressing the micro touch button (8).

The red LEDs (17', 17") are LEDs 3535 having a nominal peak wavelength of 660 nm and a nominal power of 3 W. Also the infrared LEDs (18', 18") are LEDs 3535 having a nominal peak wavelength of 805 nm and a nominal power of 3 W.

The magnifying lens (3) is an aspherical lens with +20 dioptres for indirect ophthalmoscopy (the examiner is located between the light source and the subject to be examined. Doing so, the typical red reflection of the retina, which is focused thanks to the use of special magnifying lenses, is obtained).

The device of the invention is an easy and quick to use, non-invasive instrument, with no potential health effects, and does not require the intervention of an ophthalmologic specialist. It can be realized having small size and weight, so to be a portable and handy instrument.

The device can be used in healthcare, in particular in emergency/urgency situations outside and within hospitals, such as in emergency units.

The device allows the immediate acquisition of data relating to any anomalies in oxygenation in the veins [PV-PS21] of the patient's retina. The aim is a rapid differential diagnosis (within one minute) for toxic asphyxiating systemic [PV-PS22] and potentially lethal substances, without the intervention of an ophthalmologic specialist.

Other advantages:

- pupillary dilation (pharmacological mydriasis) not required
- rapidity of diagnosis in case of oxygen deficiency, such as cyanide poisoning
- simple manufacture
- low cost
- there is no light radiation divider, nor any filter, since the sources used work at the two wavelengths necessary for the investigation, allowing to realize a relatively small and light device but on the other hand more robust than those of the known art, and therefore portable.

The device of the invention is a quick and easy to use instrument that allows, through the examination of the ocular fundus, to identify states of acute poisoning and intoxication detectable by observing morphological and chromatic variations of the retinal vessels.

In addition, the device is an instrument for non-invasive diagnosis without potential effects on human or animal health. It can be realized having a limited size and weight, so to be a portable and handy instrument.

The device can be used in healthcare, in particular in emergency/urgency situations, such as in emergency units.

**Claims**

1. A portable device for determining the oxygenation level in a human subject for the rapid diagnosis of poisoning by enzymatic toxic substances, such as cyanide, by means of an exam of an ocular fundus thereof by means of an indirect ophthalmoscopy technique associated with a camera, said device comprising:

- a non-transparent, preferably cylindrical casing housing the following items:
- a first light source (18', 18") to emit light at a wavelength of 805 nm and illuminate said ocular fundus,
- a second light source (17', 17"), separated and distinct from said first light source, to emit light at a wavelength of 660 nm and illuminate said ocular fundus
- a camera (4) arranged to acquire images of the ocular fundus when illuminated by one of said first and second light sources,
- elaborating means (9, 19) configured
- to control said first and second light sources and said camera (4) to acquire respectively first and a second images and
- to apply an algorithm to the image acquired with red light at 660nm, based on the analysis of the intensity profile of circular areas around the optical disc, to identify the positions of retinal vessels on the image and to identify (32) a list of sections of vessels and to classify them into sections of veins or arteries, and
- to calculate for each of said first and second images and for each vessel section previously identified the respective optical densities at said two different wavelengths $OD_{805}$ and $OD_{660}$ as a logarithm of the ratio between the intensity $I_0$ of the pixels detected near the section of said veins or arteries and the intensity I of the pixels detected on the section itself and to calculate an ODR ratio of the optical densities at the two wavelengths as follows $ODR = OD_{660}/OD_{805}$ to determine a level of oxygenation in the identified retinal veins.

2. The device according to claim 1, wherein said elaborating means (9, 19) are configured to acquire said first and second image by firstly activating said first light source and then said second light source.

3. The device according to claim 2, wherein said elaborating means (9,19) are configured to acquire said first and second images in a time interval shorter than 230 ms.

4. The device according to any one of claims 1 - 3, further comprising:

- a rubber eyepiece (1) at an opening of the casing, destined to face an eye to be examined;
- a magnifying lens (3) for indirect ophthalmoscopy arranged inside the casing at said opening;
- a linear actuator (11) configured to move away/closer a set consisting of said lens (3) and said camera (4) from said eye, wherein said elaborating means are configured to control said linear actuator to perform an automatic focusing on the base of images acquired by said camera during an activation of only said first light source.

5. The device according to claim 4, further comprising:

- a man/machine interface device configured for displaying information, including an oxygenation level calculated by said elaborating means (9, 19);
- a power source, such as a battery pack (5);
- a first electronic card (7) configured to load the power source (5) with an external power supply via a connector (21);
- a slide microswitch (20) configured to connect the power source (5) with the second electronic card (19);
- a second electronic card (9) configured for detecting the residual charge of the power source (5) and for adjusting the voltage supplied by the power source (5);
- a push-button (8) configured for starting the measurement of the determination of the oxygenation level.

6. The device according to any one of claims 1-5, wherein said elaborating means (9, 19) include a single-board computer (9), said computer (9) comprising an SD memory card (10) and preferably also

- a second printed circuit (19) configured for power distribution, the control of the LEDs (17', 17", 18', 18") and the control of the linear actuator (11).

7. The device according to claim 6, wherein the output device configured for displaying information is a display (2) that can be folded laterally, being associated with said casing by means of a hinge.

8. The device according to any of the previous claims 1 - 7, wherein said first and second light sources comprise at least a corresponding LED emitter.

**Patentansprüche**

1. Tragbare Vorrichtung zur Bestimmung des Sauerstoffgehalts eines Menschen zur schnellen Diagnose einer Vergiftung durch enzymatisch toxische Substanzen wie Cianid durch Untersuchung des Augenhintergrunds mittels einer indirekten Ophthalmoskopietechnik in Verbindung mit einer Kamera, wobei die Vorrichtung umfasst:

- ein nicht transparentes Gehäuse, vorzugsweise zylindrisch, das die folgenden Elemente beherbergt:

- eine erste Lichtquelle (18', 18") zum Emittieren von Licht mit einer Wellenlänge von 805 nm und zum Beleuchten des Augenhintergrunds,

- eine zweite Lichtquelle (17', 17"), die von der ersten Lichtquelle getrennt und verschieden ist, um Licht mit einer Wellenlänge von 660 nm zu emittieren und den Augenhintergrund zu beleuchten,

- eine Kamera (4), die so angeordnet ist, dass sie Bilder des Augenhintergrunds erfasst, wenn dieser von einer der ersten und zweiten Lichtquellen beleuchtet wird,

- Verarbeitungsmittel (9, 19), die konfiguriert sind,

- um die ersten und zweiten Lichtquellen und die Kamera (4) zu steuern, um jeweils ein erstes und ein zweites Bild zu erfassen und

- um einen Algorithmus auf das mit rotem Licht bei 660 nm aufgenommene Bild zu applizieren, basierend auf der Analyse des Intensitätsprofils kreisförmiger Bereiche um die Papille, um die Belichtung der Netzhautbehälter auf dem Bild zu identifizieren und eine Liste von Abschnitten der Behälter zu identifizieren (32) und zur deren Klassifizierung in Abschnitte von Venen und Arterien, und

- für jedes der ersten und zweiten Bilder und für jeden zuvor identifizierten Abschnitt der Behälter die jeweiligen optischen Dichten bei den beiden unterschiedlichen Wellenlängen OD805 und OD660 als Logarithmus des Verhältnisses zwischen der Intensität Io der in der Nähe des Abschnitts des genannten Abschnitts erfassten Pixel zu berechnen Venen oder Arterien und die Intensität I der auf dem Schnitt selbst erkannten Pixel und um ein ODR-Verhältnis der optischen Dichten bei den beiden Wellenlängen wie folgt zu berechnen: ODR = OD660/OD805, um den Grad der Sauerstoffanreicherung in den identifizierten Venen und Netzhäuten zu bestimmen.

2. Vorrichtung nach Anspruch 1, wobei die Verarbeitungsmittel (9, 19) so konfiguriert sind, dass sie die ersten und zweiten Bilder erfassen, indem sie zuerst die erste Lichtquelle und dann die zweite Lichtquelle aktivieren.

3. Vorrichtung nach Anspruch 2, wobei die Verarbeitungsmittel (9, 19) so konfiguriert sind, dass sie die ersten und zweiten Bilder in einem Zeitintervall von weniger als 230 ms erfassen.

4. Vorrichtung nach einem der Ansprüche 1-3, weiterhin umfassend:

- ein Gummiokular (1) an einer Öffnung im Gehäuse, das auf ein zu untersuchendes Auge gerichtet ist;

- eine Vergrößerungslinse (3) für die indirekte Ophthalmoskopie, die im Inneren des Gehäuses an der Öffnung angeordnet ist;

- einen linearen Aktuator (11), der so konfiguriert ist, dass er eine aus der Linse (3) und der Kamera (4) bestehende Baugruppe vom Auge weg bzw. zu dem Auge hin bewegt, wobei die Verarbeitungsmittel so konfiguriert sind, dass sie den linearen Aktuator so steuern, dass er automatisch auf der Grundlage von Bildern fokussiert von der Kamera nur während der Aktivierung der ersten Lichtquelle erfasst.

5. Vorrichtung nach Anspruch 4, weiterhin umfassend:

- eine Mensch-Maschine-Schnittstellenvorrichtung, die so konfiguriert ist, dass sie Informationen anzeigt, insbesondere einen von den Verarbeitungsmitteln (9, 19) berechneten Sauerstoffgehalt;

- eine Stromquelle, beispielsweise ein Batteriepack (5) ;

- eine erste elektronische Karte (7), die so konfiguriert ist, dass sie die Stromquelle (5) über einen Stecker (21) mit einer externen Stromversorgung auflädt;

- einen Schiebemikroschalter (20), der so konfiguriert ist, dass er die Stromquelle (5) mit der zweiten elektronischen Karte (19) verbindet;

- eine zweite elektronische Karte (9), die so konfiguriert ist, dass sie die Restladung der Stromquelle (5) erkennt und die von der Stromquelle (5) gelieferte Spannung anpasst;

- einen Druckknopf (8), der so konfiguriert ist, dass er die Messung der Bestimmung des Sauerstoffgehalts startet.

6. Vorrichtung nach einem der Ansprüche 1-5, wobei die Verarbeitungseinrichtung (9, 19) einen Einplatinencomputer (9) umfasst, wobei der Computer (9) vorzugsweise auch eine SD-Speicherkarte (10) umfasst

- eine zweite gedruckte Schaltung (19), die zur Stromverteilung, Steuerung der LEDs (17', 17", 18', 18") und Steuerung des Linearantriebs (11) konfiguriert ist.

7. Vorrichtung nach Anspruch 6, wobei die zur Anzeige von Informationen ausgebildete Ausgabevorrichtung ein seitlich klappbares Display (2) ist, das über ein Scharnier mit dem Gehäuse verbunden ist.

8. Vorrichtung nach einem der vorhergehenden Ansprüche 1-7, wobei die ersten und zweiten Lichtquellen mindestens einen entsprechenden LED-Emitter umfassen.

**Revendications**

1. Dispositif portable pour déterminer le niveau d'oxygénation chez un sujet humain pour la diagnose rapide d'un empoisonnement par des substances toxiques enzymatiques tel que du cianide au moyen d'un examen d'un fond oculaire de celui-ci au moyen d'une technique d'ophtalmoscopie indirecte associée à une caméra, ledit dispositif comprenant:

   - un boîtier non transparent, de préférence cylindrique, abritant les éléments suivants:
   - une première source lumineuse (18', 18") pour émettre de la lumière à une longueur d'onde de 805 nm et éclairer ledit fond oculaire,
   - une deuxième source lumineuse (17', 17"), séparée et distincte de ladite première source lumineuse, pour émettre de la lumière à une longueur d'onde de 660 nm et éclairer ledit fond oculaire,
   - une caméra (4) agencée pour acquérir des images du fond oculaire lorsqu'il est éclairé par l'une desdites première et deuxième sources lumineuses,
   - des moyens d'élaboration (9, 19) pour commander
   - lesdites première et deuxième sources lumineuses et ladite caméra (4) pour acquérir respectivement une première et une deuxième images et
   - appliquer un algoritme à l'image acquise avec de la lumière rouge à 660 nm, sur la base de l'analyse du profil d'intensité de zones circulaires autour du disque optique pour identifier l'exposition des conteneurs retinaux sur l'image et pour identifier (32) une liste des sections des conteneurs et pour les classifier dans des sections de veines et artères, et
   - pour calculer pour chacune desdites première et deuxième images et pour chaque section des conteneurs identifiée en précédence les respectives densités optiques auxdites deux longueurs d'onde différentes OD805 et OD660 comme logarithme du rapport entre l'intensité $I_o$ des pixels détectés à proximité de la section desdites veines ou artères et l'intensité I des pixels détectés sur la section elle-même et de calculer un rapport ODR des densités optiques aux deux longueurs d'onde comme suit ODR - OD660/OD805 pour déterminer un niveau d'oxygénation dans les veines retinales identifiées.

2. Dispositif selon la revendication 1, dans lequel lesdits moyens d'élaboration (9, 19) sont configurés pour acquérir lesdites première et deuxième images en activant d'abord ladite première source de lumière, puis ladite deuxième source de lumière.

3. Dispositif selon la revendication 2, dans lequel lesdits moyens d'élaboration (9, 19) sont configurés pour acquérir lesdites première et deuxième images dans un intervalle de temps inférieur à 230 ms.

4. Dispositif selon l'une quelconque des revendications 1-3, comprenant en outre :

   - un oculaire en caoutchouc (1) au niveau d'une ouverture du boîtier, destiné à faire face à un œil à examiner;
   - une lentille grossissante (3) pour ophtalmoscopie indirecte disposée à l'intérieur du boîtier au niveau de ladite ouverture;
   - un actionneur linéaire (11) configuré pour éloigner/rapprocher un ensemble constitué dudit objectif (3) et de ladite caméra (4) dudit oeil, dans lequel lesdits moyens d'élaboration sont configurés pour commander ledit actionneur linéaire pour effectuer une mise au point automatique sur le base d'images acquises par ladite caméra lors d'une activation de ladite première source lumineuse uniquement.

5. Dispositif selon la revendication 4, comprenant en outre:

   - un dispositif d'interface homme/machine configuré pour afficher des informations, notamment un niveau d'oxygénation calculé par lesdits moyens d'élaboration (9, 19);
   - une source d'alimentation, telle qu'un bloc-batterie (5) ;
   - une première carte électronique (7) configurée pour charger la source d'alimentation (5) avec une alimentation externe par un connecteur (21);
   - un micro-interrupteur à glissière (20) configuré pour connecter la source d'alimentation (5) à la deuxième carte électronique (19);
   - une deuxième carte électronique (9) configurée pour détecter la charge résiduelle de la source d'alimentation (5) et pour ajuster la tension fournie par la source d'alimentation (5);
   - un bouton poussoir (8) configuré pour lancer la mesure de la détermination du niveau d'oxygénation.

6. Dispositif selon l'une quelconque des revendications 1-5, dans lequel lesdits moyens d'élaboration (9, 19) comprennent un ordinateur monocarte (9), ledit ordinateur (9) comprenant une carte mémoire SD (10) et de préférence également

   - un deuxième circuit imprimé (19) configuré pour la distribution d'énergie, le contrôle des LED (17', 17", 18', 18") et le contrôle de l'actionneur linéaire (11).

7. Dispositif selon la revendication 6, dans lequel le dispositif de sortie configuré pour afficher des informations est un afficheur (2) rabattable latéralement, en étant associé audit boîtier au moyen d'une charnière.

8. Dispositif selon l'une quelconque des revendications précédentes 1-7, dans lequel lesdites première et deuxième sources lumineuses comprennent au moins un émetteur LED correspondant.

Fig. 1

Fig. 2

10

21

5

8

Fig. 3

1

Fig. 4

Fig. 5

Fig. 6

Fig. 7

Fig. 8

Fig. 9

Fig. 10

Fig. 11

Fig. 12

Fig. 13

Fig. 14

Fig. 14( continuation)

```
                    ┌─────────┐
                    │  Start  │
                    └────┬────┘
                         │                    24
                    ┌────▼─────────────────┐
                    │ Infrared LED on (805 nm) │
                    └────┬─────────────────┘
                         │                    25
                    ┌────▼─────────────────┐
                    │   Autofocusing       │
                    └────┬─────────────────┘
                         │                    26
                    ┌────▼─────────────────┐
                    │  First image acquisition │
                    └────┬─────────────────┘
                         │                    27
                    ┌────▼─────────────────┐
                    │ Infrared LED off (805 nm) │
                    └────┬─────────────────┘
                         │                    28
                    ┌────▼─────────────────┐
                    │   Red LED on (660 nm)  │
                    └────┬─────────────────┘
                         │                    29
                    ┌────▼─────────────────┐
                    │ Second image acquisition │
                    └────┬─────────────────┘
                         │                    30
                      ◇ Are images blurred? ◇
                         │
                        NO
                         │                    31
                    ┌────▼─────────────────┐
                    │   Image regulation   │
                    └────┬─────────────────┘
                         │                    32
                    ┌────▼─────────────────┐
                    │ Recognition of vein sections in │
                    │          images      │
                    └────┬─────────────────┘
                         │                    33
                    ┌────▼─────────────────┐
                    │ Calculation of oxygen │
                    │ saturation level in veins │
                    └────┬─────────────────┘
                         │                    34
                    ┌────▼─────────────────┐
                    │ Determination of patient's │
                    │    poisoning state   │
                    └────┬─────────────────┘
                         │                    35
                    ┌────▼─────────────────┐
                    │ Communication of diagnosis to │
                    │  operator via display │
                    └──────────────────────┘
```

Fig. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 102028477 A

- US 2018336679 A1, Richard M. Farchione

### Non-patent literature cited in the description

- **JONA VALGERDUR KRISTJANSDOTTIR et al.** Choroidal Oximetry With a Noninvasive Spectrophotometric Oximeter. *INVESTIGATIVE OPTHALMOLOGY & VISUAL SCIENCE,* 07 May 2013, vol. 54 (5), 3234
- **PECH-PACHECO, J.L. et al.** *Diatom Autofocusing in Brightfield Microscopy: a Comparative Study. ICPR,* 2000, http://doi.org/10.1109/ICPR.2000.903548
- **O. BERGAMIN.** *Invest. Ophthalmol. Vis. Sci.,* 2003, vol. 44 (4), 1546-1554, http://doi.org/10.1167/iovs.02-0468

- Differentiation of Artery and Vein in Digital Fundus Photograph. **KIM H. et al.** European Conference of the International Federation for Medical and Biological Engineering. IFMBE Proceedings. Springer, 2011, vol. 37
- **JÓNA VALGERÐUR KRISTJÁNSDÓTTIR.** *Choroidal and retinal oximetry,* 1985, 20-23, http://hdl.handle.net/1946/17137
- **JÓNA VALGERÐUR KRISTJÁNSDÓTTIR.** *Choroidal and retinal oximetry,* 1985, http://hdl.handle.net/1946/17137
- **THORUNN S. et al.** *Invest. Ophthalmol. Vis. Sci.,* 2012, vol. 53 (14), 2172, https://doi.org/10.1167/iovs.12-9912